# EUROPÄISCHE PATENTANMELDUNG

(11) **EP 1 103 228 A1**
(43) Veröffentlichungstag der Anmeldung: **30.05.2001**
(21) Anmeldenummer: 00125670.0
(22) Anmeldetag: 23.11.2000
(51) Int. Cl.: A61B 18/14

(54) **Gerät zur Behandlung von Gefässdefekten**

(30) Priorität: 25.11.1999 DE 19956764
(71) Anmelder: Hoffmann, Steffen, Dr., 01309 Dresden (DE)
(72) Erfinder: Hoffmann, Steffen, Dr., 01309 Dresden (DE)
(74) Vertreter: Stippl, Hubert, Dipl.-Ing.

(57) **Zusammenfassung**

Die Erfindung betrifft ein Gerät zur Behandlung von Gefäßdefekten, insbesondere Varizen unter Einsatz von Hochfrequenzenergie mit einem Gehäuse, einem Hochfrequenzgenerator sowie einem Behandlungsinstrument, welches mit dem Hochfrequenzgenerator verbunden ist und mit dem die Behandlung an dem Gefäß durchführbar ist, wobei als Behandlungsinstrument eine in das Gefäß einzubringende Sonde vorgesehen ist, die einen langgezogenen, flexiblen Sondenkörper und einen an dessen einem Ende befindlichen Sondenkopf zur Beaufschlagung des Gefäßes mit Hochfrequenzenergie umfaßt, der Sondenkörper keine Ansteuerung zur willkürlichen Bewegung des Sondenkopfs oder der Spitze des Sondenkörpers aufweist und die Flexibilität des Sondenkörpers derart festgelegt ist, daß die Sonde beim Einschieben derselben in das Gefäß dem Verlauf des Gefäßes folgend einschiebbar ist, als Zusatz zu DE 197 37 965.6, wobei der Sondenkörper (3) eine erste Querflexibilität A sowie eine zweite Querflexibilität B aufweist, wobei die Querflexibilität A größer als die Querflexibilität B ist.

## Beschreibung

Die vorliegende Erfindung betrifft ein Gerät zur Behandlung von Gefäßdefekten, insbesondere Varizen (d. h. Krampfadern) unter Einsatz von Hochfrequenzenergie nach dem Oberbegriff der Ansprüche 1 sowie 11. Ferner betrifft die Erfindung gemäß Anspruch 9 einen Sondenkörper zur Verwendung in einem Gerät zur Behandlung von Varizen.

Varizen wurden bisher vor allem, wenn es sich um Varizen größeren Durchmessers handelt, operativ entfernt, dergestalt, daß der behandelnde Arzt jeweils Schnitte entlang der Varize legt und das erkrankte Gefäß abschnittsweise über die Schnitte entfernt. Hierbei besteht das Problem, daß eine derartige Behandlung für den Patienten einerseits sehr belastend ist, zum anderen durch die Schnitte bedingte Narben bleiben.

Darüber hinaus gibt es bereits Geräte zum Schneiden oder Koagulieren von Gewebe unter Verwendung von Hochfrequenzenergie. Hierbei wird mit einer starren Nadel gearbeitet, mit der beispielsweise die Haut gestochen und ein vergleichsweise kurzer Abschnitt einer Varize behandelt wird. Bei Varizen ist es allerdings notwendig, das Gefäß über größere Längen zu behandeln, was bei der Anwendung der vorstehenden Behandlungsmethode dazu führt, die Nadel immer wieder neu einzustechen. Die Behandlung erfolgt hierbei unter

Lokalanästhesie, welche dazu führt, daß sich die im Bereich der Lokalanästhesie befindlichen Blutgefäße reflexartig zusammenziehen können. Hierbei besteht das Problem, daß bei einem erneuten Einstechen der Nadel es sehr schwierig wird, das Gefäß richtig anzustechen.

Aus der DE 197 37 965 C1 ist ein Gerät zur Behandlung von Varizen bekannt, bei dem ein langgezogener flexibler Sondenkörper ohne Ansteuerung zur willkürlichen Bewegung des Sondenkopfs bzw. der Spitze des Sondenkörpers in das Gefäß eingeführt wird, wobei dei Flexibilität des Sondenkörpers derart festgelegt ist, daß die Sonde beim Einschieben derselben in das Gefäß dem Verlauf des Gefäßes folgend einschiebbar ist. Hierbei kann es - vor allem bei sehr vulnerablen und stark gewundenen Gefäßen - dazu kommen, daß der Sondenkörper ab einer bestimmten Position nicht weiter in das Gefäß eingeschoben werden kann bzw. beim weiteren Einschieben Verletzungen der Gefäßwand auftreten.

Die Aufgabe der vorliegenden Erfindung besteht darin, ein verbessertes Gerät zur Behandlung von Gefäßdefekten, insbesondere Varizen zur Verfügung zu stellen, mit dem eine patientenschonendere Behandlung vor allem auch bei sehr feinen Gefäßen vorgenommen werden kann.

Die vorstehende Aufgabe wird bei dem gattungsgemäßen Gerät dadurch gelöst, daß der Sondenkörper eine erste Querflexibilität A sowie eine zweite Querflexibilität B aufweist, wobei die Querflexibilität A größer ist als die Querflexibilität B. Die Flexibilität des Sondenkörpers ist somit anisotrop, d. h. der Sondenkörper läßt sich in der ersten Querflexibilität A zugeordneten

Richtung leichter biegen als in der der Querflexibilität B zugeordneten Richtung. Der Sondenkörper gewährleistet somit eine unterschiedliche Biegesteifigkeit. Bleibt der Sondenkörper bei der Behandlung beispielsweise hängen, kann der behandelnde Arzt den Sondenkörper leicht zurückziehen und gleichzeitig unter einer Drehbewegung den Sondenkörper entsprechend den Verlauf der Varize weiter einführen. Die Suche nach der geeigneten Stellung der Spitze des Sondenkörpers bzw. Sondenkopfs erfolgt sehr viel schneller als unter Verwendung der bisher zur Verfügung gestandenen Technik. Demzufolge nimmt das Risiko von Verletzungen der Gefäßwände stark ab.

Zur Erzielung der unterschiedlichen Querflexibilität weist der Sondenkörper zweckmäßigerweise eine Strukturierung auf, die entweder für eine gezielte Erhöhung oder Erniedrigung der Querflexibilität sorgt.

Als Strukturierung kann zweckmäßigerweise eine Wellung oder fortlaufende Knickung des Sondenkörpers, z. B. des Drahts, vorgesehen sein, wodurch der Draht in der Ebene der Wellung bzw. Knickung eine kleinere Querflexibilität aufweist als beispielsweise in einer Ebene senkrecht zur Wellung oder Knickung.

Die unterschiedliche Querflexibilität kann über die gesamte Länge des Sondenkörpers vorgesehen sein, so daß der Sondenkörper einfacherweise aus endlos Drahtmaterial, welches beispielsweise mit einer Strukturierung versehen ist, einfacherweise produziert werden kann.

Alternativ kann es allerdings auch vorgesehen sein, den Sondenkörper mit unterschiedlichen Querflexibilitäten A sowie B lediglich über einen Teil seiner Gesamtlänge auszustatten.

Weiterhin besteht eine zweckmäßige Ausgestaltung darin, durch Variierung der Strukturierung eine Variierung der Querflexibilität in einer Richtung beispielsweise durch Erhöhung der Frequenz der Wellung bzw. Knickung vorzusehen.

Ferner ist eine zweckmäßige Ausgestaltung der Erfindung dadurch gekennzeichnet, daß der Sondenkopf als Schlaufe ausgebildet ist. Dies kann einfacherweise durch Schlaufenbildung des Endstücks des Sondenkörpers erfolgen. Die Schlaufe hat den Vorteil, daß der Sondenkörper im Gefäß nicht steckenbleiben kann und dadurch ungewollte Beschädigungen der Gefäßwände ausgeschlossen werden.

Der Gegenstand des Anspruchs 7 hat den Vorteil, daß die Führung, mit der der Sondenkörper von außen durch die Haut in das Gefäß eingebracht werden kann, kleinstmögliche Abmessungen aufweisen kann.

Sofern die Schlaufe ungefüllt ist, entstehen im Inneren der Schlaufe hohe Temperaturen, die zu einer Koagulation des darin befindlichen Blutes führen und damit eine Thrombosierung des Gefäßes verursachen könnten. Bei therapeutischer Zielsetzung des vollständigen Verschlusses der Varize ist dies jedoch ohne Belang.

Ist lediglich eine Reduzierung des Gefäßdurchmessers, d. h. eine Verbesserung der Funktion des Gefäßes gewünscht, ist die Schlaufe vorzugsweise gefüllt und zwar entweder flach gefüllt oder weist eine tropfenförmige Füllung auf.

Gegenstand der vorliegenden Erfindung ist weiterhin ein Sondenkörper als erfindungswesentlich funktionalisiertes Element des vorerwähnten Gerätes nach einem der Ansprüche 1 - 9.

Zweckmäßigerweise kann das erfindungsgemäße Gerät, auch nebengeordnet beansprucht, gemäß Anspruch 11 derart ausgestaltet sein, daß der Sondenkopf zusätzlich zur Erfassung der Temperatur im Gefäß dient, so daß der behandelnde Arzt die Temperatur, d. h. Hitzeentwicklung an der betreffenden Stelle stets kontrollieren kann und in Abhängigkeit davon den Sondenkörper innerhalb des Gefäßes führen kann bzw. die Energie auf das Gefäß verändern kann.

Gemäß einer Ausgestaltung der vorliegenden Erfindung erfolgt sowohl die Temperaturerfassung als auch die Beaufschlagung mit Hochfrequenzenergie über den Sondenkörper bzw. Sondenkopf. Die Temperaturerfassung erfolgt hierbei zweckmäßigerweise durch Aufnahme der Erwärmung des Sondenkörpers. Hierzu ist der Sondenkörper aus gut wärmeleitfähigem Material herzustellen.

Weiterhin ist es vorteilhaft, eine Umschalteinrichtung vorzusehen, die zwischen Hochfrequenzbeaufschlagung sowie Temperaturmessung umschaltet. Diese Umschalteinrichtung kann mit einem vom behandelnden Arzt individuell einstellbaren Zeitintervall für die Hochfrequenzbeaufschlagung versehen sein.

Zum Legen der Sonde ist vorteilhafterweise eine Führung (insbesondere eine sogenannte "Venenverweilkanüle") vorgesehen, die vorzugsweise mit einer Stichpositionskontrolle, d.h. einer Kontrolle zur Überprüfung, ob das Gefäß angestochen worden ist, ausgeführt sein kann. Eine Führung ist allerdings nicht immer notwendig. Ohne Führung kann die Sonde während einer Operation gelegt werden (z.B. bei Crossektomie der Vena saphena magna).

Um Beschädigungen des umliegenden, gesunden Gewebes zu vermeiden, ist gemäß einer weiteren Ausgestaltung der vorliegenden Erfindung die Temperatur im Bereich des Sondenkopfes erfaßbar.

Der Sondenkörper besteht hierbei aus temperaturleitfähigem Material, zumindest aber umfaßt er solches. Alternativ hierzu kann der Sondenkörper auch eine temperaturleitfähige Beschichtung aufweisen.

Besondere Ausgestaltungen der vorliegenden Erfindung werden anhand der Zeichnungen nachstehend näher erläutert. Es zeigen:
- Fig. 1: eine stark vereinfachte, schematische Darstellungsweise des Gerätes gemäß dem Stand der Technik in seiner Gesamtheit im Einsatz;
- Fig. 2: eine Teildarstellung des zum Einsatz des Gerätes zu verwendenden Sondenkörpers;
- Fig. 3: eine alternative Ausgestaltung eines Sondenkörpers;
- Fig. 4: eine Seitendarstellung des Sondenkörpers gemäß Fig. 3 mit Füllung;
- Fig. 5: eine stirnseitige Ansicht des Sondenkörpers gemäß Fig. 4 mit flacher Füllung sowie
- Fig.6: eine stirnseitige Ansicht des Sondenkörpers gemäß Fig. 4 mit tropfenförmiger Füllung.

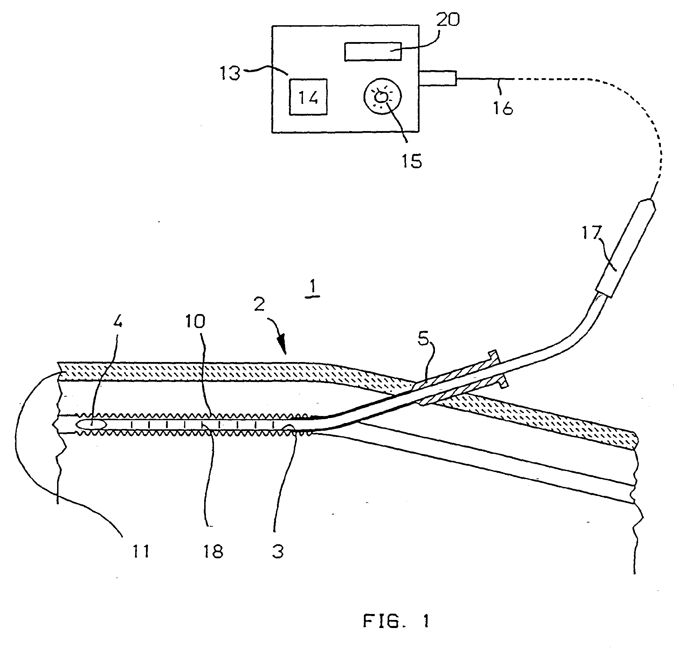

Bezugsziffer 1 in Fig. 1 zeigt das gattungsgemäße Gerät gemäß DE 197 379 65 C1 in seiner Gesamtheit. Auf den Inhalt von DE 197 37 965 C1 wird vollinhaltlich Bezug genommen. Das Gerät umfaßt eine flexible, langgezogene Sonde 2 mit einem Handgriff 17, einen z.B. aus Wolfram oder Stahl bestehenden Sondenkörper 3 sowie einem am Ende des Sondenkörpers 3 angeordneten Sondenkopfs 4 z. B. aus Au, PT oder Ag zur Abgabe von Hochfrequenzenergie.

Der Sondenkörper 3 ist über eine Führung 5 durch die Haut in das erkrankte Gefäß 10 eingeführt. Bezugsziffer 11 in Fig. 1 bezeichnet die verschiedenartigen Hautschichten des menschlichen Körpers.

Die Sonde steht über ein Kabel 16 mit der Hochfrequenzquelle 14 bzw. den Gehäuse 13 desselben in Verbindung. Die Hochfrequenzquelle 14 weist darüber hinaus eine Anzeige 20 sowie eine Verstelleinrichtung 15 zur Abgabe der Hochfrequenzenergie auf.

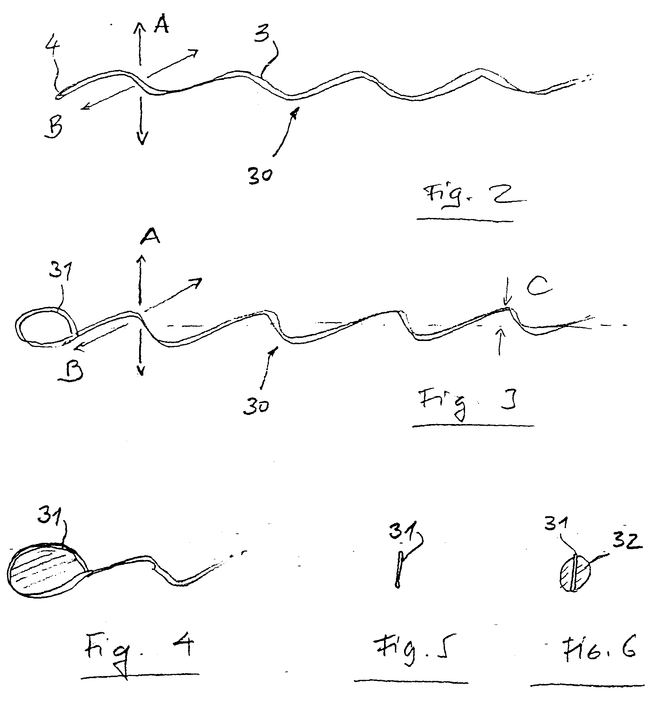

Die Führung umfaßt gemäß Fig. 2 eine Hohlnadel 6 mit einem Hohlraum 8, welcher als Stichpositionskontrolle dient. Bei richtig plaziertem Stich füllt sich der Hohlraum 8 mit Blut. Die Führung 5 ist dergestalt ausgebildet, daß nach dem Setzen der Ummantelung 7 in der Haut die Nadel entfernt und der Sondenkörper eingeführt werden kann.

Der Sondenkörper 3 weist einen innenliegenden Metalldraht 12, eine den Metalldraht umgebende Kunststoff-, insbesondere Teflonbeschichtung 9 sowie ggf. eine temperaturleitfähige Beschichtung auf. Gemäß den Fig. 3 b) und c) erstreckt sich die temperaturleitfähige Beschichtung lediglich über einen Teil des Umfangsbereichs des Sondenkörpers. Über die temperaturleitfähige Schicht 19 kann in einfacher Weise die Temperatur am Sondenkopf 4 "gefühlt" werden und an der Anzeige 20 angezeigt werden. Auch ist es möglich, bei Überschreiten einer bestimmten Temperatur ein (nicht dargestelltes) Warnsignal zu aktivieren.

In bestimmten Fällen, insbesondere bei sehr dünnen Sonden, ist es zweckmäßig, den Metalldraht aus dem identischen Material wie den Sondenkopf herzustellen, d.h. diese beiden Teile sozusagen in einem Teil zur Verfügung zu stellen. Der Sondenkopf wird hierbei durch die nicht von der beschichteten Zone abgedeckte Zone des Metalldrahts gebildet.

Zur Behandlung einer Varize wird die Führung über deren Nadel durch die Haut 11 in das zu behandelnde Gefäß 10 eingebracht und die Nadel anschließend entfernt. Über einen Hohlraum an der Nadel wird sichtbar, daß das zu behandelnde Gefäß tatsächlich auch getroffen worden ist. Anschließend wird die Sonde durch die Führung 5 hindurch in das behandelnde Gefäß über die gesamte Behandlungslänge hinweg eingeschoben. Die Dimensionierung von Sondenkopf 4 sowie Sondenkörper 3 einschließlich Führung 5 bewirken eine automatische Abdichtung der Führung 5.

Soll in Lokalanästhesie gearbeitet werden, reicht es aus, die Punktionsstelle mit Lokalanästhesie-creme vorzubehandeln. Nach dem Legen der Sonde kann dann entlang der Varize ein Lokalanästhetikum injiziert werden. Sollen mehrere Varizen behandelt werden, ist es vorteilhaft, in Kurznarkose (5 min.) mehrere Sonden parallel zu legen und Tumeszenzlokalanästhesie zu injizieren.

Da vor dem Legen der Sonde der Patient narkotisiert wird, ist es zweckmäßig, während einer Behandlung gleichzeitig eine Mehrzahl von Sonden zur Behandlung einer Mehrzahl von Gefäßen zu legen. Sind die Sonden gelegt, wird das jeweilige Gefäß möglichst blutleer gemacht (z.B. durch Hochlegen, Anlegen einer Blutsperre oder von Kompressionswickel). Danach wird das betreffende Gefäß unter dem Einfluß von Hochfrequenzenergie (beispielsweise im Megahertzbereich) behandelt, wobei die Gefäßwände hierbei miteinander verschweißen, d.h. koagulieren oder sich der Gefäßdurchmesser verringert. Während der Behandlung wird die Sonde vom entlegensten zum Punktionspunkt in dem Gefäß allmählich aus dem Gefäß herausgezogen, wodurch eine fortlaufende Verschweißung der Gefäßwände stattfindet. An den Hochfrequenzgenerator selbst kann, beispielsweise bei Überschreiten einer bestimmten Temperatur, die Hochfrequenzenergie über einen Einstellknopf 15 (vergleiche Fig. 1) verändert werden.

Abhängig von der jeweils zu behandelnden Varize können pro Gerät unterschiedlich lange oder auch unterschiedlich starke Sondenkörper 3 eingesetzt werden.

An der Vorderseite des Sondenkörpers 3 befindet sich der Sondenkopf 4 in Form einer plattgedrückten Kugel. Der Sondenkörper 3 besteht z.B. aus Metalldraht oder Wolfram. Es sind allerdings auch andere Materialien hierfür als denkbar anzusehen.

Gemäß der vorliegenden Erfindung ist der Sondenkörper 3 mit einer ersten Querflexibilität A (vgl. Fig. 2) sowie einer zweiten Querflexibilität B ausgestattet, wobei die Querflexibilität A größer sein soll als die Querflexibilität B. Dies kann zweckmäßigerweise in einfacher Realisierung dadurch ermöglicht werden, daß der Sondenkörper 3 eine Strukturierung, zweckmäßigerweise eine Wellung 30 oder fortlaufende Knickung aufweist. Hierdurch wird in der Ebene der Wellung 30, die der Richtung der Querflexibilität A entspricht, eine höhere Querflexibilität erreicht im Vergleich zur Querflexibilität B, welche beispielsweise senkrecht zu der der Querflexibilität A zugeordneten Ebene liegt. Der Sondekörper 3 besteht aus einem sehr dünnen Metalldraht, z. B. Wolframdraht, welcher bis auf den Sondenkopf 4 mit einer (nicht dargestellten) Isolierung versehen ist. Die Wellung 30 ist zweckmäßigerweise fortlaufend über die gesamte Länge des Sondenkörpers 3 ausgeführt, so daß dieser in einfacher Weise als Endlosteil produziert werden kann. Bei der in Fig. 2 dargestellten Ausgestaltung des Sondenkörpers ist der Sondenkopf 4 lediglich als von der Isolierung nicht erfaßte Spitze des Drahts ausgebildet.

Demgegenüber ist bei der in Fig. 3 dargestellten Ausführungsform an der Vorderseite, d. h. im Bereich des Sondenkopfs 4 eine Schlaufe 31 bestehend aus nicht isoliertem Metalldraht vorgesehen. Zweckmäßigerweise weist die Schlaufe - in Querrichtung zur Längsachse des Sondenkörpers 3 gesehen - einen Durchmesser auf, welcher etwa kleiner oder gleich der doppelten Amplitude C der Wellung 30 ist. Die Schlaufe 31 ist vorzugsweise geschlossen, um Verletzungen der Gefäßwand beim Zurückziehen des Sondenkörpers 3 zu vermeiden.

Aus Fig. 4 ist eine weitere Ausgestaltung der vorliegenden Erfindung zu erkennen, bei der das Innere der Schlaufe 31 gefüllt ist, um zu vermeiden, daß die Temperatur im Inneren der Schlaufe 31 bei der Behandlung zu hoch wird.

Das Innere der Schlaufe 31 kann, wie in Fig. 5 dargestellt, durch eine flächige Füllung, beispielsweise hergestellt durch Eintauchen der Schlaufe 31 in ein Metallbad, versehen sein.

Alternativ hierzu kann auch eine tropfenförmige Füllung 32 vorgesehen sein, wie dies aus Fig. 6 ersichtlich ist.

Das erfindungsgemäße Gerät kann je nach den gegebenen Einsatzbedingungen mit den vorerwähnten Sondenkörpern 3 ausgestattet werden.

Weiterhin ist vorgesehen, daß der Sondenkopf 4 zusätzlich zur Erfassung der Temperatur im Gefäß 10 dient. Die Temperaturerfassung erfolgt zweckmäßigerweise über die Erwärmung des Sondenkopfs 4 sowie Sondenkörpers 3 während der Beaufschlagung mit Hochfrequenzenergie. Alternativ hierzu ist es auch denkbar, über ein am Sondenkopf 4 vorgesehenes Mikrothermoelement die Temperatur im Gefäß 10 zu erfassen.

Eine Besonderheit der Erfindung besteht darin, daß eine Umschalteinrichtung im Gerät vorgesehen ist, die zwischen Hochfrequenzbeaufschlagung sowie Temperaturmessung umschaltet, d. h. einen intermittierenden Betrieb ermöglicht. Die Umschalteinrichtung ist mit einer Zeitintervallschaltung ausgestattet, mit der das Zeitintervall der Hochfrequenzbeaufschlagung festgelegt wird. Die Zeitintervallschaltung ist vom behandelnden Arzt individuell einstellbar. Eine Überschreitung der Behandlungstemperatur wird dadurch verhindert, daß die Umschaltung von der Temperaturmessung in den Hochfre quenzbetrieb erst nach Erreichen der optimalen Arbeitstemperatur erfolgt.

### BEZUGSZEICHENLISTE

- 1: Gerät (komplett)
- 2: Sonde
- 3: Sondenkörper
- 4: Sondenkopf
- 5: Führung
- 6: Nadel
- 7: Ummantelung
- 8: Hohlraum
- 9: Kunststoffummantelung
- 10: Gefäß
- 11: Haut
- 12: Metalldraht
- 13: Gehäuse
- 14: Frequenzgenerator
- 15: Einstellknopf
- 16: Kabel
- 17: Handgriff
- 18: Skalierung
- 19: leitfähige Beschichtung
- 20: Temperaturanzeige
- 21: Führungshülle
- 30: Wellung
- 31: Schlaufe
- 32: Füllung

## Patentansprüche

1. Gerät zur Behandlung von Gefäßdefekten, insbesondere Varizen unter Einsatz von Hochfrequenzenergie mit einem Gehäuse, einem Hochfrequenzgenerator sowie einem Behandlungsinstrument, welches mit dem Hochfrequenzgenerator verbunden ist und mit dem die Behandlung an dem Gefäß durchführbar ist, wobei als Behandlungsinstrument eine in das Gefäß einzubringende Sonde vorgesehen ist, die einen langgezogenen, flexiblen Sondenkörper und einen an dessen einem Ende befindlichen Sondenkopf zur Beaufschlagung des Gefäßes mit Hochfrequenzenergie umfaßt, der Sondenkörper keine Ansteuerung zur willkürlichen Bewegung des Sondenkopfs oder der Spitze des Sondenkörpers aufweist und die Flexibilität des Sondenkörpers derart festgelegt ist, daß die Sonde beim Einschieben derselben in das Gefäß dem Verlauf des Gefäßes folgend einschiebbar ist, als Zusatz zu DE 197 37 965.6,
**dadurch gekennzeichnet, daß**
der Sondenkörper (3) eine erste Querflexibilität A sowie eine zweite Querflexibilität B aufweist, wobei die Querflexibilität A größer als die Querflexibilität B ist.

2. Gerät nach Anspruch 1,
**dadurch gekennzeichnet, daß**
der Sondenkörper (3) zur Erzielung der Querflexibilität A sowie B eine Strukturierung aufweist.

3. Gerät nach Anspruch 2,
**dadurch gekennzeichnet, daß**
als Strukturierung eine Wellung (30) oder fortlaufende Knickung vorgesehen ist.

4. Gerät nach einem der vorhergehenden Ansprüche,
**dadurch gekennzeichnet, daß**
der Sondenkörper (3) über seine gesamte Länge unterschiedliche Querflexibilitäten A sowie B aufweist.

5. Gerät nach einem der vorhergehenden Ansprüche 1 - 3,
**dadurch gekennzeichnet, daß**
der Sondenkörper (3) über einen Teil seiner Gesamtlänge unterschiedliche Querflexibilitäten A sowie B aufweist.

6. Gerät nach einem der vorhergehenden Ansprüche,
**dadurch gekennzeichnet, daß**
als Sondenkopf (4) eine Schlaufe (31) vorgesehen ist.

7. Gerät nach Anspruch 6,
**dadurch gekennzeichnet, daß**
der Durchmesser der Schlaufe (31) - in Querrichtung zur Längsachse des Sondenkörpers (3) gesehen - etwa kleiner oder gleich der doppelten Amplitude C der Wellung (30) oder fortlaufenden Knickung des Sondenkörpers (3) ist.

8. Gerät nach Anspruch 6 oder 7,
**dadurch gekennzeichnet, daß**
die Schlaufe (31) gefüllt ist.

9. Gerät nach Anspruch 8,
**dadurch gekennzeichnet, daß**
die Schlaufe (31) flach gefüllt ist oder eine tropfenförmige Füllung (32) aufweist.

10. Sondenkörper (3) zur Verwendung in einem Gerät zur Behandlung von Gefäßdefekten, insbesondere Varizen unter Einsatz von Hochfrequenzenergie nach einem der vorstehenden Ansprüche 1 - 9.

11. Gerät zur Behandlung von Gefäßdefekten, insbesondere Varizen unter Einsatz von Hochfrequenzenergie, insbesondere nach einem der vorhergehenden Ansprüche 1 - 9, mit einem Gehäuse, einem Hochfrequenzgenerator sowie einem Behandlungsinstrument, welches mit dem Hochfrequenzgenerator verbunden ist und mit dem die Behandlung an dem Gefäß durchführbar ist, wobei als Behandlungsinstrument eine in das Gefäß einzubringende Sonde vorgesehen ist, die einen langgezogenen, flexiblen Sondenkörper und einen an dessen einem Ende befindlichen Sondenkopf zur Beaufschlagung des Gefäßes mit Hochfrequenzenergie umfaßt, der Sondenkörper keine Ansteuerung zur willkürlichen Bewegung des Sondenkopfs oder der Spitze des Sondenkörpers aufweist und die Flexibilität des Sondenkörpers derart festgelegt ist, daß die Sonde beim Einschieben derselben in das Gefäß dem Verlauf des Gefäßes folgend einschiebbar ist,
**dadurch gekennzeichnet, daß**
der Sondenkopf (4) zusätzlich zur Erfassung der Temperatur im Gefäß (10) dient.

12. Gerät nach Anspruch 11,
**dadurch gekennzeichnet, daß**
die Temperaturerfassung über die Erwärmung des Sondenkopfs (4) sowie Sondenkörpers (3) während der Beaufschlagung mit Hochfrequenzenergie erfolgt.

13. Gerät nach Anspruch 11 oder 12,
**dadurch gekennzeichnet, daß**
eine Umschalteinrichtung vorgesehen ist, die zwischen Hochfrequenzbeaufschlagung sowie Temperaturmessung umschaltet.

14. Gerät nach Anspruch 13,
**dadurch gekennzeichnet, daß**
das Zeitintervall der Hochfrequenzbeaufschlagung individuell einstellbar ist.
